# EUROPÄISCHE PATENTANMELDUNG

(11) **EP 2 719 371 A2**
(43) Veröffentlichungstag der Anmeldung: **16.04.2014**
(21) Anmeldenummer: 13002555.4
(22) Anmeldetag: 15.05.2013
(51) Int. Cl.: A61K 8/34, A61K 8/84, A61Q 13/00

(54) **Alkoholfreies 'Kölnisch Wasser'-Ersatzprodukt**

(30) Priorität: 15.05.2012 DE 102012010206
(71) Anmelder: Kaynak, Koray, 38114 Braunschweig (DE)
(72) Erfinder: Kaynak, Koray, 38114 Braunschweig (DE)
(74) Vertreter: Lins, Martina

(57) **Zusammenfassung**

Das ,Kölnisch Wasser'-Ersatzprodukt stellt ein herkömmliches ,Kölnisch Wasser' aus Wasser, hochprozentigem Ethanol und Duftstoffen nach, um dessen leicht antiseptische, erfrischende und reinigende Wirkung in einem ethanolfreien Produkt nutzen zu können. Hierfür enthält das 'Kölnisch Wasser'-Ersatzprodukt Duftstoffe in einer wasserbasierten Zusammensetzung unter Ausschluss von alkoholischen Lösungsmitteln, wie insbesondere Ethanol und Isopropanol, und zwar
- eine Duftstoffkomponente,
- eine Glycolkomponente
- wenigstens ein Antiseptikum
- wenigstens ein amphoteres, neutrales oder kationisches Tensid in einer Menge, die gerade ausreicht, die übrigen Bestandteile ohne Phasentrennung in der wässrigen Zusammensetzung zu solubilisieren.

Das ,Kölnisch Wasser'-Ersatzprodukt kann in jeder für 'Kölnisch Wasser' üblichen Verwendung eingesetzt werden, auch zum Tränken von Erfrischungstüchern und innerhalb von kosmetischen Erzeugnissen.

## Beschreibung

Die Erfindung betrifft ein ,Kölnisch Wasser'-Ersatzprodukt, das Duftstoffe in einer wasserbasierten Zusammensetzung und das dabei keine alkoholischen Lösungsmittel, wie insbesondere Ethanol und/oder Isopropanol, enthält.

Ein 'Kölnisch Wasser' ist ein im Vergleich zu anderen Duftwassertypen leicht parfümiertes Duftwasser und Erfrischungsmittel, das auf den berühmten Kölner Parfümeur Johann Maria Farina zurückgeht. Dieser schuf im Jahre 1709 aus bestimmten Duftölen, die u.a. eine Zitruskomponente enthielten, sowie aus sehr hochprozentigem Alkohol ein Duftwasser. Zu Ehren seiner Heimatstadt Köln nannte er es "Eau de Cologne". Dieses Duftwasser wird noch heute von seinen Nachfolgern in unveränderter Rezeptur hergestellt und weltweit erfolgreich verkauft. Inzwischen gibt es zahlreiche ,Kölnisch Wasser'-Produkte, die sich in ihren Duftnoten unterscheiden. Die Bezeichnung ,Kölnisch Wasser' gibt daher heute in erster Linie Auskunft über den Riechstoffgehalt. Man unterscheidet:

| | |
|---|---|
| Kölnisch Wasser (Eau de Cologne) | bis zu 5 %ig |
| Eau de Toilette | 3-10 %ig |
| Eau de Parfüm | 8-15 %ig |
| Parfüm | 15-30 %ig, |

wobei die Angaben den Duftstoffanteil/Duftölanteil in Bezug auf das Duftwasser-Gesamtgewicht angeben. Es handelt sich um Angaben zwecks grober Klassifizierung, die Werte können nach oben und unten abweichen. ,Kölnisch Wasser' wird aufgrund seines hohen Alkoholgehalts und seiner sparsameren Parfümierung sowie des Charakters der darin meist verwendeten Duftnoten u.a. als erfrischend und belebend beworben. Es kann reichlicher verwendet werden als die stärker parfümierten Duftwässer oder gar Parfüms.

Der relativ hohe Alkoholgehalt - es handelt sich bei dem Alkohol um Lösemittelalkohole, speziell Monoalkanole und insbesondere Ethanol - trägt durch Eigengeruch zur Gesamtduftnote bei und besitzt darüber hinaus antiseptische bzw. desinfizierende Wirkung. Zusätzlich dient der Alkoholgehalt zum Lösen der häufig wasserunlöslichen Duftstoffe bzw. Duftöle, bewirkt eine gute Verdunstungsfähigkeit auf Oberflächen, wie der Haut, transportiert die Duftstoffe in die Umgebungsluft und bewirkt so den gewünschten Parfümierungseffekt. Durch die Verdunstung des Alkohols trocknet gleichzeitig das Produkt auf, falls dies auf der Haut des Benutzers geschieht, sollte sich ein gereinigtes und nicht nasses Gefühl ergeben. Ein klassisches ,Kölnisch Wasser' besteht fast ausschließlich aus Wasser, Alkohol und der Duftstoffkomponente - einer durch Parfümeure bestimmten Mischung.

Aufgrund der vorgenannten Wirkungen ist ,Kölnisch Wasser' in den südlichen Ländern, u.a. Südeuropa und dem Vorderen Orient sehr weit verbreitet und aus dem täglichen Gebrauch nicht wegzudenken. Seit mehr als 100 Jahren ist das bewährte ,Kölnisch Wasser' unter dem Namen "Kolonya" in der türkischen und orientalischen Kultur stark verwurzelt. In der Türkei ist "Kolonya" bei der Erfrischung und Reinigung bis heute bewährt und das mit großem Abstand meist verkaufte Duftwasser auf Alkoholbasis. Fast jeder türkische Haushalt ist mit "Kolonya" ausgestattet. Auch in öffentlichen Einrichtungen, Hotels und Restaurants findet in der Türkei und im Orient stets vor der Begrüßung ein ritualisiertes Reinigen und Erfrischen der Hände mit "Kolonya" statt. Bei einem türkischen Friseur bzw. Barbier wird "Kolonya" reichlich zur Desinfektion und Hautreinigung verwendet. Es hat sich dort nachweislich als meist eingesetztes Hautreinigungsmittel in der täglichen Anwendung etabliert.

Der hohe Alkoholgehalt der ,Kölnisch Wasser'-Produkte bringt allerdings auch verschiedene Nachteile mit sich. Die häufige und reichliche Verwendung von Alkohol auf der Haut kann zu Hautirritationen und Ausschlägen führen. Der Alkohol hat Einfluss auf die Fett- und Feuchtigkeitsregulation der Haut und kann so die Haut austrocknen, was wiederum Allergien fördert und den natürlichen Eigenschutz der Haut schwächt. Ein großes Problem in muslimisch geprägten Gegenden besteht außerdem darin, dass Muslime aus Glaubensgründen keinen Alkohol in ihren Produkten verwenden sollten. Es besteht daher ein Bedarf an einem für Muslime geeigneten ,Kölnisch Wasser' mit Halal-Zertifizierung.

Der Erfindung liegt daher die Aufgabe zugrunde, die Probleme im Stand der Technik zu lösen und ein ,Kölnisch Wasser'-Ersatzprodukt zu schaffen, das den Geboten der islamischen Regeln für Sauberkeit und Reinheit genügt, kein Ethanol enthält und das in Erscheinungsbild und Haptik einem ,Kölnisch Wasser' möglichst gut nachgestellt ist.

Diese Aufgabe wird durch ein ,Kölnisch Wasser'-Ersatzprodukt gemäß Anspruch 1, ein zugehöriges Erfrischungstuch nach Anspruch 7 und ein kosmetisches Erzeugnis nach Anspruch 8 gelöst. Das ,Kölnisch-Wasser'-Ersatzprodukt enthält Duftstoffe in einer wasserbasierten Zusammensetzung und keine alkoholischen Lösungsmittel, nämlich keine Monoalkanole und insbesondere kein Ethanol und Isopropanol. Das ,Kölnisch Wasser'-Ersatzprodukt ist durch folgende Inhaltsstoffe gekennzeichnet:
- eine Duftstoffkomponente, die vorzugsweise ein oder mehrere Bestandteile aus der Gruppe natürliche oder synthetische Duftstoffe, Parfümöle, ätherische Öle, insbesondere Pflanzenöle, Extrakte, Destillate oder andere duftende Essenzen enthält;
- eine Glycolkomponente, die wenigstens ein Glycol ausgewählt aus der Gruppe Ethylenglycol, Propylenglycol, Ethoxydiglycol, Polyethylenglycole und Polyethylenglycol-Konjugate und als Konjugat vorzugsweise pegyliertes hydriertes Rizinusöl enthält;

- wenigstens ein Antiseptikum;
- wenigstens ein amphoteres, neutrales oder kationisches Tensid in einer Menge, die gerade ausreicht, die übrigen Bestandteile ohne Phasentrennung in der wässrigen Zusammensetzung zu solubilisieren.

Durch die Erfindung wird ein herkömmliches ,Kölnisch Wasser' aus Wasser, hochprozentigem Alkohol (Ethanol) und Duftstoffen nachgestellt, um dessen leicht antiseptische, erfrischende und reinigende Wirkung in einem ethanolfreien Produkt nutzen zu können.

Das erfindungsgemäße ,Kölnisch Wasser'-Ersatzprodukt enthält erfindungsgemäß ca. 1 bis 7 Gew.% der Duftstoffkomponente bezogen auf das Gesamtprodukt. Da der Einfluss des Alkohols auf die Duftwirkung fehlt, kann der Gehalt etwas höher sein als bei einem üblichen, traditionellen ,Kölnisch Wasser'. Auch die Glycolkomponente mischt sich jedoch in den Geruch ein, sodass die Parfümierung überdeckt bzw. beeinträchtigt aber auch verstärkt werden kann. Entsprechend kann die Untergrenze für den Duftstoffanteil durch gegenseitige Geruchsverstärkung auch geringer ausfallen als bei einem traditionellen ,Kölnisch Wasser'.

Die Duftstoffe werden grundsätzlich, wie bei jeder Parfümierung, so zusammengestellt, wie vom Kunden gewünscht. Der ,Kölnisch Wasser'-Charakter sollte dabei vorzugsweise erhalten bleiben, was jedoch für die Wirkung des neuen Ersatzprodukts selbstverständlich nicht ausschlaggebend ist. Duftstoffe und Duftstoffmischungen sind im Handel erhältlich.

Die Glycolkomponente wird zur Solubilisierung der Duftstoffkomponente in der Wasserbasis eingesetzt. Wasserbasiert bedeutet, dass Wasser das Hauptlösungsmittel darstellt; das ,Kölnisch Wasser'-Ersatzprodukt ist grundsätzlich eine wässrige Lösung, d.h. Wasser ist der Bestandteil mit dem größten Gewicht in der Zusammensetzung. Die gewählten Glycole haben lösungsvermittelnde bis emulgierende Eigenschaften. Sie sind regelmäßig in jedem Verhältnis mit Wasser mischbar und verändern dabei u.a. die Polarität und die Löseeigenschaften. Die Wirkungsweise der Glycole aus der Glycolkomponente ist grundsätzlich eine Lösungsvermittlerwirkung. Als Polyethylenglycol-Konjugate können hier solche verwendet werden, die auch als Emulgatoren bekannt sind, bevorzugt sind Polyethylenglycol-Konjugate mit Fetten oder Ölen, insbesondere pegyliertes hydriertes Rizinusöl und ganz besonders hydriertes Rizinusöl-PEG40.

Es hat sich herausgestellt, dass die Glycole optimale Lösungsvermittler für die Duftstoffe in einer rein wässrigen Basis darstellen. Sie tragen wesentlich zum Nachstellen der ,Kölnisch Wasser'-Eigenschaften in Bezug auf das Hautgefühl bei. Die Glycole gleichen das Fehlen der Monoalkanole bzw. alkoholischen Lösemittel aus. Sie dienen gleichzeitig zur Herstellung einer klaren Lösung, aus der sich die Duftstoffe nicht in einer eigenen Phase absetzen (Aufschwimmen, Blasenbildung). Zwar soll die Bildung einer Mikroemulsion hier nicht ausgeschlossen werden, es soll sich dabei jedoch nicht um eine stark trübende oder opaleszierende Emulsion handeln. Durch die Emulgatoreigenschaften bestimmter Glycole können insbesondere Duftöle, allgemein ölhaltige oder fetthaltige Essenzen oder sonstige hydrophobe Duftstoffe sehr fein emulgiert werden, dies sollte sich jedoch nach Möglichkeit im Bereich sehr feiner Emulsionen, also von Mikroemulsionen und Nanoemulsionen (mizellarer Bereich) abspielen und die optische Erscheinungsform des Produktes nicht beeinträchtigen.

Das erfindungsgemäße ,Kölnisch Wasser'-Ersatzprodukt sollte bei üblicher Verwendung, also beispielsweise bei Verteilen auf den Händen, nicht schäumen und nicht merkbar aufemulgieren.

Der Anteil an hydrophoben und hydrophilen Komponenten in der Duftstoffkomponente ist von der jeweiligen von einem Parfümeur zusammengestellten Formulierung abhängig, was wiederum den für die Wirkung erforderlichen Glycolgehalt beeinflusst. Es hat sich herausgestellt, dass das Verhältnis der Duftstoffkomponente zur Glycolkomponente vorteilhafterweise zwischen 3 zu 7 und 1 zu 9 beträgt (Gewichtsverhältnis).

Die Glycolkomponente besteht vorzugsweise aus einem Gemisch aus mehreren Glycolen, wobei wiederum vorzugsweise reine Glycole und Glycolether einerseits und konjugierte Glycole, nämlich in der Kosmetikindustrie bekannte Emulgatoren dieser Klasse, andererseits im Verhältnis zwischen ca. 2 zu 1 und 1 zu 2 gemischt vorliegen (Gewichtsverhältnisse).

Besonders bevorzugt ist es, wenn die Glycolkomponente aus einem Gemisch aus Ethoxydiglycol, Propylenglycol und PEG40-konjugiertem hydriertem Rizinusöl besteht.

Duftstoffkomponente und Glycolkomponente nehmen gemeinsam vorzugsweise nicht mehr als 50 Gew.% der Zusammensetzung, d.h. des erfindungsgemäßen Ersatzproduktes, ein, weiter vorzugsweise nicht mehr als 20 Gew.% und besonders bevorzugt nicht mehr als 12 Gew.%.

Die Formulierung enthält wenigstens ein Antiseptikum, um die desinfizierende Wirkung des Alkohols im traditionellen ,Kölnisch Wasser' ersetzen und sogar übertreffen zu können. Unter einem Antiseptikum wird ein Desinfektionsmittel verstanden, das auf der Haut, nämlich mit antiseptischer Wirkung, verwendet werden kann. Antiseptika bzw. hierfür geeignete Desinfektionsmittel haben im Allgemeinen bakterizide oder zumindest bakteriostatische, allgemein mikrobielle und vorzugsweise virustatische Eigenschaften. Grundsätzlich können alle für die Hautdesinfektion verwendeten Substanzen hier eingesetzt werden, wie sie beispielsweise aus Hautinfektionsmitteln für den Privatbereich oder im Klinikbetrieb, für medizinische Seifen u. dgl. bekannt sind. Eine Konzentration von maximal 3 Gew.%, vorzugsweise maximal 1 Gew.% ist ausreichend. Eingesetzt werden können beispielsweise Polyhexabiguanid (PHBG), insbesondere Polyhexanid = Poly(hexamethylenbiguanid)hydrochlorid (PHMB), Benzalkoniumchlorid, Triclosan, Cetylpyridiniumchlorid, Oxichinolin und andere mehr, Mischungen der vorgenannten Stoffe untereinander oder Mischungen eines der vorgenannten Stoffe mit einem anderen Hautdesinfektionsmittel. Im Rahmen dieser Erfindung ist der Einsatz von Polyhexanid bevorzugt, das relativ geruchsneutral ist, farblos bis schwach gelb, von geeignetem pH-Wert zwischen 5 und 6 und sich daher in die Rezeptur gut einfügt. Bevorzugt wird weniger als 1 Gew.% PHMB eingesetzt.

Die Zusammensetzung für das ,Kölnisch Wasser'-Ersatzprodukt umfasst weiterhin wenigstens ein amphoteres, neutrales oder kationisches Tensid in einer Menge, die gerade ausreicht, die übrigen Bestandteile ohne Phasentrennung in der wässrigen Zusammensetzung zu solubilisieren. Der Zusatz eines sehr milden Tensids in geringer Menge dient nochmals der Solubilisierung, d.h. Lösungsvermittlung zwischen den Duftstoffen, die hydrophob sein können, und dem grundsätzlich wässrigen Medium. Da das neue ,Kölnisch Wasser'-Ersatzprodukt vorzugsweise zu wenigstens 80 bis ca. 97 Gew.% aus Wasser besteht, sind zum Nachstellen des traditionellen Alkoholgemischs verschiedene Maßnahmen erforderlich. Die Solubilisierung der Duftstoffe wird im ersten Schritt bereits durch die Glycole bewerkstelligt, mit denen die Duftstoffkomponente vorzugsweise vorgemischt vorliegt. Eine sehr geringe Menge eines milden Tensids bewirkt nun, dass die Haptik nochmals verbessert wird, das Duftwasser sich insgesamt weicher und leichter beweglich anfühlt. Das zusätzliche Tensid verhindert auch bei Lagerung über längere Zeit, dass sich eine Phasentrennung einstellen kann und sich die Duftkomponente absondert, was unerwünscht wäre.

Die Tensidmenge ist so einzustellen, dass der gewünschte Effekt gerade eintritt, was im Allgemeinen mit Tensidkonzentrationen unter 1 Gew.%, vorzugsweise unter 0,5 Gew.%, weiter vorzugsweise unter 0,3 Gew.% ohne weiteres möglich ist.

Das ,Kölnisch Wasser'-Ersatzprodukt soll bei gewöhnlichem Gebrauch, beispielsweise Verteilen auf den Händen und Verreiben, weder erkennbar aufemulgieren, noch schäumen. Haptisch und optisch soll sich annähernd die Wirkung eines ,Kölnisch Wasser'-Produkts ergeben. Hierfür ist eine gute Duftfreisetzung und ein erfrischendes Hautgefühl Voraussetzung.

Als Tenside sind im Rahmen dieser Erfindung alle milden kosmetischen Tenside geeignet, insbesondere Zuckertenside, aminosäurebasierte Tenside, die in der Regel amphoter oder kationisch wirken, insbesondere Glycintenside und/oder Betain-Tenside. Die Tenside werden einzeln oder im Gemisch eingesetzt. Geeignet sind sowohl Mischungen innerhalb der genannten Tenside als auch Mischungen der genannten Tenside mit weiteren Tensiden, soweit dies nicht anionische Tenside oder stark basische Tenside sind.

Es ist erwünscht, dass der pH-Wert der Gesamtmischung einen Wert zwischen 6,2 und 6,6 einnimmt. Allgemein sollte der pH-Wert vorzugsweise ca. zwischen 5 und 7,5 liegen. Falls es gewünscht ist, den pH-Wert zu korrigieren, kann dies beispielsweise durch Zugabe von Zitronensäure geschehen oder durch andere milde und in geringer Menge hautverträgliche Säuren oder Basen.

Die Duftstoffkomponente des neuen ,Kölnisch Wasser'-Ersatzprodukts wird nach Bedarf zusammengestellt. Die Duftnote bestimmt durch die Ausgangsstoffe auch den hydrophoben bzw. hydrophilen Charakter der Duftstoffmischung. Klassische Duftbestandteile eines ,Kölnisch Wassers' enthalten Zitrusnoten, sodass eine Mischung mit Zitrusnote auch hier besonders bevorzugt ist. Duftstoffkomponenten mit Zitrusnote enthalten häufig hohe Anteile an Lemonen, Linalool, Citral, Geraniol, Citronellal. Andere zum Zitrusfruchtspektrum gehörende Duftsubstanzen können einzeln oder in verschiedenen Zusammenstellungen hinzukommen. Außerdem sind weitere Ergänzungen durch Aromen und/oder Parfümöl, beispielsweise Bergamotte, möglich. Selbstverständlich sind auch andere Duftkreationen möglich, beispielsweise können blumige Düfte allein oder in Kombination mit Zitrusdüften zum Einsatz kommen, ebenso andere fruchtige Duftnoten, Gewürz- oder Kräuteressenzen, z.B. Myrrhe, Minze u.a.. Auch grüne Düfte, wie Vetiver kommen in Frage.

Mögliche Duftstoffe, auch als Riechstoffe bezeichnet, sind dem Fachmann, d.h. dem Aromatiseur oder Parfümeur bekannt und müssen hier nicht im Einzelnen ausgeführt werden. Natürliche Duftstoffe sind bevorzugt. Gerade die Zitronennoten werden durch natürliche oder naturidentische Aromen am besten wiedergegeben. Die Duftstoffkomponente kann daher aus wenigstens einem der folgenden Bestandteile bestehen: aus Duftölen, vorzugsweise ätherischen Ölen natürlichen Ursprungs oder Auszügen daraus, aus synthetischen Duftstoffen, wobei diese vorzugsweise nur mit natürlichen Duftstoffen zusammen verwendet werden, aus Duftstoffen tierischen Ursprungs, wie Amber oder Moschus, aus Extrakten, insbesondere Pflanzenextrakten einschließlich Kräuterextrakten, aus Destillaten, auch Wasserdampfdestillaten, aus Mazeraten oder aus verschiedenen Essenzen, d.h. Duftstoffkonzentraten verschiedener Herstellungsform und Herkunft.

Bevorzugte Duftstoffkomponenten werden häufig einen Duftöl- bzw. Parfümölanteil besitzen, da viele Düfte in Öl gewonnen werden, von Natur aus Öle enthalten oder in diesen bezüglich ihres Dufts gut konservierbar sind.

Eine Zitrusnote kann beispielsweise Zitronenöl enthalten, das einen hohen Bestandteil an Limonene enthält. Limonene ist zu über 90 % in Zitronenöl und Orangenöl enthalten. Dabei werden Zitrusdüfte sehr stark von Terpenen und Terpenoiden, insbesondere zyklischen und azyklischen Monoterpenen Terpenaldehyden und -estern bestimmt. Zu den Inhaltsstoffen für Zitronenduftnoten zählen u.a. das vorher schon genannte Limonene, außerdem Citral, Geraniol, γ-Terpenen, Linalool, Citronellal, Campher, Sinesal und Nerylactetat.

Das erfindungsgemäße ,Kölnisch Wasser'-Ersatzprodukt besitzt zahlreiche Vorteile. Es ist antimikobiell, dermatologisch und allergologisch getestet sowie ha-Ial. Es ermöglicht die Reinigung und Pflege der Haut ohne die irritierende Wirkung des Ethanols oder Isopropanols. Es besitzt eine hygienisierende Wirkung ohne Beschädigung der aktiven Barrierefunktion der Haut oder bleibende Beeinträchtigung der Hautflora. Das neue Produkt brennt nicht auf der Haut und kann langfristig verwendet werden, wobei die Gefahr von Allergien und Exzemen herabgesetzt ist. Eine desodorierende Wirkung wird durch die keimreduzierende Wirkung des Antiseptikums erreicht.

Das Produkt dient auch den durch die Weltgesundheitsbehörde (WHO) geforderten Bedingungen einer hygienischen Reinigung von Händen und Füßen. Die WHO hat alle Muslime, die Pilger- und Wallfahrten nach Mekka vornehmen, aufgefordert, sich auf ihren Reisen zum Schutz ihrer eigenen Gesundheit regelmäßig die Hände und Füße nachhaltig zu reinigen. Ein hierfür geeignetes halal-zertifiziertes Produkt ist mit dem neuen ,Kölnisch Wasser'-Ersatzprodukt nun verfügbar.

Das erfindungsgemäße ,Kölnisch Wasser'-Ersatzprodukt ist daher vielfältig verwendbar und kann für alle traditionellen Verwendungsformen des ,Kölnisch Wassers' genutzt werden.

Die Erfindung umfasst daher auch Produkte, bei denen das ,Kölnisch Wasser'-Ersatzprodukt ebenso wie bisherige ,Kölnisch Wasser' eingesetzt werden kann. Sämtliche kosmetischen Erzeugnisse können mit dem erfindungsgemäßen ,Kölnisch Wasser'-Ersatzprodukt wie mit einem herkömmlichen Eau de Cologne parfümiert werden.

Die Erfindung umfasst auch ein Erfrischungstuch, das mit dem ,Kölnisch Wasser'-Ersatzprodukt nach der Erfindung getränkt ist.

Im Folgenden wird die Erfindung anhand von Ausführungsbeispielen und Rezepturen in Verbindung mit weiteren Produktangaben näher erläutert.

### Beispiel 1 Grundrezeptur mit Zitrusnote

| **Inhaltsstoff** | **Gew.%** |
|---|---|
| Duftstoffkomponente mit Glycolkomponente vorvermischt, insgesamt | 1-10 |
| Antiseptikum VANTOCIL TG® (Arch UK Biocides Ltd.) | < 1 |
| 30 %ige wässrige Lösung eines Undecylensäureamidopropyldimethylcarboximethylammoniumbetains als Tensid, REWOTERIC AM B U 185® (EVONIK Industries, DE) | < 1 |
| Wasser | ad 100 |

Dabei bestand die eingesetzte Mischung aus Duftstoffkomponente und Glycolkomponente aus:

| **INCl-Bezeichnung** | **Gew.%** |
|---|---|
| PEG-40 Hydrogenated Castor Oil | 25-50 |
| Ethoxydiglycol | 10-25 |
| Propylene glycol | 10-25 |
| Duftstoffkomponente | 8-30 |
| | (insgesamt 100 Gew.%) |

Anstelle des Tensids REWOTERIC AM B U 185 können andere milde Tenside für kosmetische Formulierungen eingesetzt werden, vorzugsweise andere Tenside aus der Gruppe "REWOTERIC®".

Das Antiseptikum VANTOCIL TG® kann entsprechend durch andere Antiseptika ausgetauscht werden, falls gewünscht.

Sofern gewünscht und erforderlich, kann der pH-Wert der Zusammensetzung mit Zitronensäure auf pH 6,2 bis 6,6 eingestellt werden.

Für die Parfümierung wurde eine Zitrus-Duftmischung verwendet, die u.a. Parfüm (Fragance), Limonene und Citral enthielt.

### Angaben zu den Rohstoffen:

### Wasser: gereinigtes Trinkwasser

REWOTERIC®-Tensid AM B U 185: Undecylenamidopropylbetain, sehr mildes Tensid, hoch rein, eingesetzt als klare wässrige Lösung (30 %ig), pH-Wert der 30 %igen wässrigen Tensidlösung 4,5 bis 6, Viskosität < 100 mPas (Brookfield), Toxizität (Ratte) LD 50 > 2000 mg/kg
VANTOCIL TG® (Antiseptikum): Poly(hexamethylenbiguanid)hydrochlorid = "Polyhexanid" schnell wirkendes Bakterizid, farblos bis gelblich, klar, hautverträglich, nicht karzinogen, Toxizität (Ratte) LD 50 > 2000 mg/kg
PEG-40 hydriertes Rizinusöl (Emulgator und Lösungsvermittler): CAS-61788-85-0, universeller Lösungsvermittler, leicht gelb, pH-Wert 6,2 bis 6,8, Toxizität (Ratte) LD 50 > 20 g/kg.

### Herstellung

Das ,Kölnisch Wasser'-Ersatzprodukt kann nach üblichen labortechnischen Verfahren für Duftwässer und Kosmetika hergestellt werden. Bei der Vorbereitung und Herstellung der Mischung müssen die GMP-Richtlinien eingehalten werden.

Die mit der Glycolkomponente vorgemischte Duftstoffkomponente wird zunächst mit dem Antiseptikum und dem milden Tensid vermischt und bis zur Homogenität gerührt. Das Wasser wird unter Rühren der homogenen Mischung zugegeben. Nach Abschluss der Wasserzugabe wird noch 30 Minuten weitergerührt, und es werden pH-Wert und Dichte gemessen.

Sofern der pH-Wert mit Hilfe von Zitronensäure auf einen Wert von 6,2 bis 6,6 eingestellt wird, wird danach noch wenigstens 10 Minuten gerührt.

Die Dichte soll einen Wert von 1,003 bis 1,005 g/ml besitzen.

### Gutachten und Prüfberichte

- antimikrobielle Wirkung gemäß DIN EN 1276-2009 bestätigt
- Epikutan-Test - dermatologisch-allergologische Verträglichkeit durch fachärztliches Gutachten bestätigt
- Wirksamkeit gegen Influenzaviren H1/N1 entsprechend DIN EN 14476: 2005 - 08 bestätigt
- halal-zertifiziert durch IGB e.V. Braunschweig.

## Patentansprüche

1. Kölnisch Wasser'-Ersatzprodukt, das Duftstoffe in einer wasserbasierten Zusammensetzung enthält, **gekennzeichnet durch** folgende Inhaltsstoffe:
- eine Duftstoffkomponente;
- eine Glycolkomponente, die wenigstens ein Glycol ausgewählt aus der Gruppe Ethylenglycol, Propylenglycol, Ethoxydiglycol, Polyethylenglycole und Polyethylenglycol-Konjugate enthält;
- wenigstens ein Antiseptikum;
- wenigstens ein amphoteres, neutrales oder kationisches Tensid in einer Menge, die gerade ausreicht, die übrigen Bestandteile ohne Phasentrennung in der wässrigen Zusammensetzung zu solubilisieren;
unter Ausschluss von alkoholischen Lösungsmitteln, wie insbesondere Ethanol und Isopropanol, wobei der Anteil der Duftkomponente im Produkt 1 bis 7 Gew.-% beträgt und das Verhältnis der Duftstoffkomponente zur Glycolkomponente zwischen 3:7 und 1:9 beträgt.

2. Kölnisch Wasser'-Ersatzprodukt nach Anspruch 1, **dadurch gekennzeichnet, dass** die Glycolkomponente aus einem Gemisch aus Ethoxydiglycol, Propyplenglycol und PEG40-konjugiertem hydriertem Rizinusöl besteht.

3. Kölnisch Wasser'-Ersatzprodukt nach Anspruch 1 oder 2, **dadurch gekennzeichnet, dass** das Antiseptikum ausgewählt ist aus der Gruppe Polyhexabiguanid, insbesondere Polyhexanid, Benzalkonium, Triclosan, Cetylpyridiniumchlorid, Oxichinolin, einzeln oder im Gemisch.

4. Kölnisch Wasser'-Ersatzprodukt nach einem der Ansprüche 1 bis 3, **dadurch gekennzeichnet, dass** das Tensid ausgewählt aus der Gruppe Zuckertenside, aminosäurebasierte Tenside, insbesondere Glycintenside, Betain-Tenside, die einzeln oder im Gemisch enthalten sind.

5. Kölnisch Wasser'-Ersatzprodukt nach einem der Ansprüche 1 bis 4, **dadurch gekennzeichnet, dass** die Duftstoffkomponente eine Mischung mit Zitrusnote ist.

6. Kölnisch Wasser'-Ersatzprodukt nach einem der Ansprüche 1 bis 5, **dadurch gekennzeichnet, dass** die Duftstoffkomponente aus wenigstens einem der folgenden Bestandteile besteht: Duftöle, vorzugsweise ätherische Öle natürlichen Ursprungs oder Auszüge daraus, synthetische Duftstoffe, Duftstoffe tierischen Ursprungs, Extrakte, insbesondere Pflanzenextrakte, Destillate, Mazerate, Essenzen, natürliche Duftstoffe, wie insbesondere Terpene, Terpenoide, Terpenole, Terpenaldehyde und -ester.

7. Erfrischungstuch, getränkt mit dem ,Kölnisch Wasser'-Ersatzprodukt nach einem der Ansprüche 1 bis 6.

8. Kosmetisches Erzeugnis, parfumiert mit dem ,Kölnisch Wasser'-Ersatzprodukt nach einem der Ansprüche 1 bis 6.
